# EUROPEAN PATENT SPECIFICATION

(11) **EP 0 925 031 B1**
(45) Date of publication and mention of the grant of the patent: **10.09.2003**
(21) Application number: 98928473.2
(22) Date of filing: 15.06.1998
(51) Int. Cl.: A61B 17/12, A61B 17/00

(54) **MEDICAL DEVICE**
MEDIZINISCHES GERÄT
APPAREIL MEDICAL

(30) Priority: 18.06.1997 GB 9712707; 24.02.1998 GB 9803846
(43) Date of publication of application: 30.06.1999
(73) Proprietor: Hudson, John, Glenfield, Leicester LE3 8AG (GB); Bauer, Alberto, St. Petersburg Beach, FL 33706 (US)
(72) Inventor: Hudson, John, Glenfield, Leicester LE3 8AG (GB); Bauer, Alberto, St. Petersburg Beach, FL 33706 (US)
(74) Representative: Thomas, Philip John Duval
(86) International application number: GB9801732
(87) International publication number: WO98057586

(56) References cited:
- WO-A-94/06460
- US-A- 4 638 803
- US-A- 5 224 497
- US-A- 5 308 326

## Description

The present invention relates to an apparatus for plugging a diverticulum.

Diverticula are sacral pockets or protrusions of variable size occurring normally through a defect in the muscular coating of a tubular organ, such as the intestinal tract.

Diverticula of the intestinal tract derive from the lumen and extend through the wall of the gut and are believed to be a contributory factor in colonic dysfunction. Typically, they may be enclosed by all layers of the bowel (serosa, muscle and mucosa), termed "true" diverticula; or they may lack muscle as a component of the sac, termed "false" or "pseudo" diverticula. True diverticula are usually congenital, while false diverticula are acquired, either by disease or secondary to pulsation forces in the gut, and typically are found at a site of an artery which feeds the bowel wall.

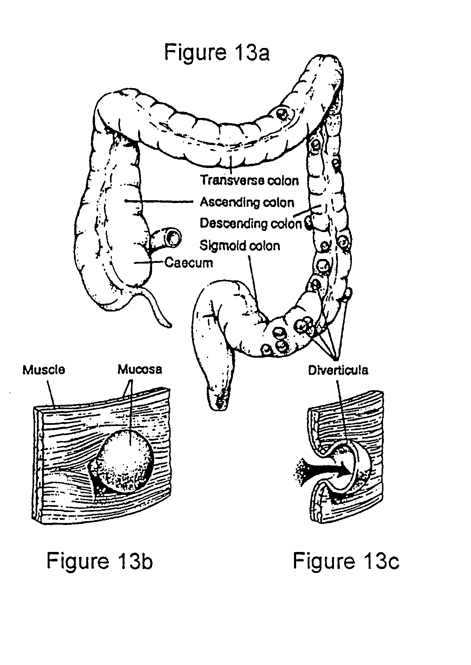

As shown in Figure 13, the diverticula of the colon are usually multiple and may be accompanied by inflammation. In the absence of inflammation the condition is known as diverticulosis, while in the presence of inflammation the condition is known as diverticulitis. Although the distinction between the two conditions is clear when a colon is examined surgically or histologically, the symptoms of colonic dysfunction due to diverticulosis sometimes mimic those of diverticulitis and, therefore, the clinical distinction between the two conditions may often be blurred. Consequently, diverticular disease of the colon typically refers to all stages of the disease from diverticulosis.

In diverticulosis the diverticula are initially microscopic in size but peristaltic contractions may cause them to enlarge slowly, until they become globular nodules projecting from the external surface of the bowel. These globular nodules are typically about one centimetre in diameter, though "giant diverticula" of over 3 centimetres in diameter are not unknown.

The globular diverticula communicate with the bowel lumen via a narrow neck through which gas can pass freely. They are prone to filling with faecal material extruded from the lumen that may then become firm. Such a process usually begins in the Sigmoid area and then typically spreads proximally throughout the intestinal tract over the course of several years. Infection and inflammation of a diverticulum results in diverticulitis, and such inflammation occurs in approximately 15 to 25 percent of patients with diverticulosis.

Complications associated with diverticular disease include sepsis, fistula. bleeding, obstruction and intractable painful disturbance of the bowel function, with typical septic complications being abscess and perforation accompanied with diffuse bacterial and/or feculent peritonitis.

One of the most dangerous complications of diverticular disease is that of bleeding diverticula.

Diverticula usually form at a site where an artery supplying blood to mucosa penetrates the muscular wall of the colon. The penetration of the muscular wall causes a "weak point" and a diverticulum may form, with the result that the artery terminates in the mucosa inside the diverticulum.

If the diverticulum becomes infected, or if compacted faecal matter causes an irritation, then the end of the artery may fail causing excessive bleeding. As this bleeding is arterial it is generally very difficult to control, and major surgery involving forming a colostomy and, a few months later, reconnecting the colon or removal of a section of the colon is often the only available treatment.

Although the true global prevalence of the disease is unknown, it has been estimated that in western countries the disease is uncommon before the age of forty but approximately seventy percent of people may have the disease by the age of eighty five.

Accordingly, there is a need not only for a method of treating diverticular disease which prevents inflammation of diverticula, thereby arresting the progression of diverticulosis to diverticulitis, but also a treatment which prevents re-infection, ulceration, bleeding or perforation of previously inflamed diverticula that have been treated against infection.

Balloon devices are known in the prior art. US 5,308,326 provides a balloon tamponade device for treating bleeding sites within the upper digestive tract and comprises a tube that is sufficiently rigid to provide a passage through its inner lumen, the tube having an inner diameter of 1.5cm and an outer diameter of 2.0cm, to allow for normal swallowing and feeding functions. US 4,638,803 provides an inflatable balloon capable of being delivered to a site within the body and includes on its surface a composition having sufficient concentration of metallic thrombosis-producing material to insure that the metallic thrombosis reaction will occur. US 4,638,803 recommends that metallic particles of 5 microns or less are used on the surface of the balloon because they are non-toxic to the body.

The present invention seeks to provide an apparatus for the treatment of diverticular disease.

Accordingly, the invention provides apparatus comprising a delivery means and a balloon connected to the delivery means characterised in that, when the balloon is deflated, the deflated balloon has a diameter dimensioned to permit entry through the neck of a diverticulum and wherein the balloon is associated with a non-metallic haemostatic agent that retards or prevents bleeding.

Preferably, the balloon is releasably connected to the delivery means and includes means to prevent deflation.

The term "non-metallic" is intended to embrace materials which do not have metallic properties. For example, the term "non-metallic" is intended to include a material that is a poor conductor of electricity and a material that preferably forms negative ions in an aqueous solution or in combination with other elements, such as metals. The term "non-metallic" is not intended to include metals such as iron, copper, tantalum, and the noble metals, gold, silver and platinum, chronium and their derivatives including oxides, carbonyl compounds and alloys such as stainless steel.

The term "a non-metallic haemostatic agent that retards or prevents bleeding" is intended to include any non-metallic agent that is capable of arresting, stemming or preventing bleeding by means other than inducing tissue growth alone. In other words, it is not tissue growth alone which is responsible for retarding or preventing bleeding. It will of course be appreciated that the non-metallic haemostatic agent may have the beneficial property of inducing tissue growth in addition to its retardation or prevention of bleeding property.

Preferably, the non-metallic haemostatic agent is a bioactive compound or composition which causes vasoconstriction and/or blood coagulation.

Examples of preferred non-metallic haemostatic agents that retard or prevent bleeding include oxidised cellulose, such as Tabotamp™ sold by Johnson and Johnson, calcium alginate, gelatine or collagen. A particularly preferred agent is carboxymethylated cellulose which can be purchased from Courtaulds Special Fibres, PO Box 111, 101 Lockhurst Lane, Coventry, England, CV6 5RS. Combinations of different agents may be used within the scope of the invention.

A particular advantage of the present invention is that the non-metallic haemostatic agent that retards or prevents bleeding exhibits a low toxicity to the human body.

Another advantage of the present invention is that a synergistic effect is producted by the combination of the non-metallic haemostatic agent that retards or prevents bleeding and the controlled pressure exerted by the balloon on a bleeding artery of a diverticulum. This synergistic effect minimises the time required to stop arterial bleeding of a diverticulum, thereby increasing patient compliance. On its own, the controlled pressure exerted by the balloon may prolong the time required to stop bleeding.

The non-metallic haemostatic agent that retards or prevents bleeding also advantageously acts as a prophylactic agent to prevent arterial bleeding of a non-bleeding diverticulum.

Preferably, the non-metallic haemostatic agent that retards or prevents bleeding is provided in the form of a net or knitted, especially a weft knitted, textile material that envelopes the balloon. More preferably, the net or knitted textile material is fixed to the balloon and/or has a roughened surface to promote growth of fibrous tissue around the outer surface of the balloon thereby anchoring it to the interior wall of the diverticulum.

Alternatively, the non-metallic haemostatic agent that retards or prevents bleeding is provided in the form of a flexible film that coats the outer surface of the balloon. Preferably, the flexible film has a roughened surface to promote tissue growth.

Conveniently, the balloon is made of an elastomeric material, such as silicone rubber.

Preferably, the balloon is made from material that is impermeable to liquid but permeable to gas so that the balloon and anti-deflation means can be tested before use by inflating the balloon with air. If there are no leaks, the balloon can be deflated by placing it in a vacuum chamber for 2 to 3 hours which causes the air to permeate through the balloon wall.

Preferably, the balloon is an inflatable balloon having an inflation port provided with a non-return valve arrangement. Such an arrangement allows the balloon to be inflated irreversibly by entry of a filler material through the inflation port.

Preferably, the non-return valve comprises a tube sealed at one end having an outlet in the side wall, the outlet being releasably sealed with a valve cover. The valve cover is preferably formed from part of the balloon, more preferably from part of the neck of the balloon.

However, it will be appreciated that if the non-metallic haemostatic agent which prevents or retards bleeding is relatively fast-acting, the balloon can be fixed to the delivery means and may not need to be provided with means to prevent deflation. The balloon can simply be pushed inside a diverticulum until the bleeding has been prevented or retarded and then removed.

Advantageously, the outer surface of the balloon is textured or roughened to increase its surface area and, in use, promote tissue growth to encapsulate the balloon and thereby close a diverticulum. To further promote the growth of tissue, a tissue growth promoting agent can be provided. For example, the outer surface of the balloon may be coated with a growth promoting agent in the form of a bio-absorbable polymer such as polyglycolic acid. Other growth promoting agents include polysaccharides, polylactic acid (PLA), polylactide, cellulose esters, polycaprolactone, polyethylene glycol, polyphosphate ester, collagen, polydioxanone (PDS), trimethylene carbonate (TMC), hyaluronic acid (HLA) and other glycosaminoglycans which are within the knowledge of a skilled person.

Preferably, diverticula which have been treated are marked by suitable means to distinguish them from untreated diverticula. Suitable marking can be achieved in a variety of ways. For example, a marker "flag" can be provided in the form of an attachment to the balloon which protrudes from the neck of a diverticulum after insertion of the apparatus. Preferably such a flag is formed of a bio-degradable material such as sodium alginate.

Alternatively or additionally, a dye can be used as a marker. The dye can be applied as a sprays or as an injection i.e. like a tattoo. Preferred dyes include 5% methylene blue; Lugol's solution; toluidene blue; 1:4 solution of washable blue ink; 0.1% indigo carmine. A suitable tattoo medium could be an aliquot of sterile indian ink, diluted 1:100. The use of dyes is within the knowledge of a skilled person.

Preferably, the marker means is visible to the naked human eye without extracorporeal detection or visualisation means, even though in practice the person performing the treatment may use such visualisation means eg an endoscope. Hence, the term "visible to the naked human eye" is not intended to embrace, for example, a radiopaque agent which is invisible to the human eye without radionuclide detection means such as a gamma camera.

Although not essential, the balloon is provided with anchor means for engaging the interior wall of the diverticulum to retain the balloon inside a diverticulum in use. Optionally, the anchor means and/or balloon may be provided with marker means.

The term "anchor means" is intended to embrace any physical means by which the balloon can be retained within a diverticulum, for example, an arrangement of one or more fingers, barbs or springs which can attach themselves onto the interior wall of the diverticulum and prevent the balloon from being expelled from the diverticulum by the peristaltic movement of the bowel.

Preferably, penetration of the interior wall of a diverticulum by an anchor is limited by one or more abutment means.

Preferably, the anchor is constructed from a shape memory alloy, a superelastic material or an elastic material, more preferably from nitinol, tempered steel, or a moulded plastic such as a thermosetting or thermoplastic plastic.

By "shape memory alloy" is meant an alloy which can be preformed into a first preferred shape while hot, cooled and folded into a second shape, the second shape remaining fixed until the alloy is heated whereupon it returns to its first preferred shape.

Use of such alloys advantageously permit the anchor to be folded from a first preferred shape into a second shape which is small enough to fit into the end of a catheter. After insertion into a diverticulum the anchor returns to its first preferred shape due to the heat of the patient's body.

By the term "superelastic material", we mean a material which has elastic properties such that a large change in strain is accompanied by a relatively small change in stress. That is, a material which has a modulus of elasticity (Young's modulus), close to zero.

The dimensions of the anchor will be selected so that in a retaining position the maximum dimensions of the anchor are greater than the maximum internal dimensions of a diverticulum i.e. from about 1 to 3 centimetres.

Preferably, the delivery means for inserting the balloon into a diverticulum is a catheter.

Preferably, the catheter has a recess for receiving the balloon a release mechanism for releasing the balloon from the catheter and, optionally, an injection means for injecting filler material into the balloon.

More preferably, the catheter comprises an inner tube that is coaxial with an outer tube, the inner tube slidably engaging the outer tube and having an injection means at a first end and a part for receiving the balloon at the first said end. The outer tube has an end portion outwardly extending from the first end of the inner tube defining the recess for receiving the balloon with the second end of the outer tube being joined to the inner tube by a seal. The release mechanism, in use, reciprocates the outer tube relative to the inner tube. In this preferred arrangement the outer tube sits over the mouth of a diverticulum and acts as a restraining brace while the balloon is inserted into the diverticulum, filled with the filler material, and the injection means removed from the balloon. This prevents application of any undue force to the inside of the diverticulum being plugged, which increases patient compliance and further accelerates the encapsulation of the balloon with body tissue.

Preferably, the outer tube includes a sheath to receive the balloon.

Preferably, the seal joining the outer tube to the inner tube has an inlet port for receiving a biocompatible fluid. This enables an Endoscopist to flush any body tissue from the recess restraining the balloon or to wash the inside of a diverticulum prior to insertion of the balloon. Alternatively, the Endoscopist may pass a dye through the inlet port and spray mark the diverticulum which has been filled. This permits the Endoscopist to quickly identify which diverticula have been filled, thus increasing the efficiency of the overall process, with a concomitant decrease in Apparatus for plugging, a diverticulum may comprise:
a filling means for insertion into the diverticulum, a delivery means releasably connected to the filling means for inserting the filling means into the diverticulum and a marker means that is visible to the naked human eye for indicating a diverticulum which has been plugged.

The term "filling means" is intended to include any material and/or apparatus which can be inserted into, and substantially fill, a diverticulum to inhibit and preferably prevent, amongst other things, the ingress of deleterious substances such as faecal matter.

Preferably, the filling means comprises an inflatable balloon.

Another preferred filling means comprises a curable material which can be used in non-solid form eg as a liquid, paste or gel form to fill a diverticulum and then cured into a hardened state.

The curable material should be bio-compatible and preferably quick-setting so that that procedure can be conducted rapidly. Suitable curable materials include silicone elastomers, polyurethane foam or elastomer, epoxy resins for example. Setting or curing of the material can be effected by solvent evaporation, ultra violet light or visible irradiation, or by heating.

The term "bio-compatible" is intended to mean being harmonious to life and not having toxic or injurious effects on biological function. This term is however, intended to embrace material which has a slightly irritant or thrombogenic effect in a host organism. Such an effect may be useful to promote tissue growth over a plugged diverticulum.

The curable material may be provided in two or more parts for mixing outside or within the diverticulum.

Preferably anchor means is inserted into a diverticulum with the curable material to retain the cured material within the diverticulum.

A further preferred filling means comprises a fibrous material such as a textile thread or fibre. Preferably the threads or fibres are coated with an adhesive material. The adhesive can be applied before, after, or at the same time as the fibrous material is inserted into a diverticulum.

Examples of suitable adhesives include Fibrin glues; non toxic thermosetting elastomeric adhesives such as moisture and UV light curing types and cyanoacrylates. Some examples may be found in the range of medically approved products distributed by Loctite UK Ltd.

Preferably, anchor means is provided to retain the fibrous material within the diverticulum.

Another preferred filling means comprises a resiliently deformable member, preferably an elastomeric member. In use, the elastomeric member is compressed, inserted into a diverticulum and released so that it expands to fill the diverticulum.

By "resiliently deformable member" we include the meaning of a member which can be deformed from a retaining shape in which it can be retained inside a diverticulum, to a smaller shape in which it can be inserted inside a diverticulum. The term "resiliently" is used to denote that the member can return to its retaining shape under suitable conditions.

Preferably, the deformable member is provided with anchor means constructed and arranged to retain the member within the diverticulum in use.

Advantageously, diverticula which have been treated are marked by a marker means that is visible to the naked human eye to distinguish them from untreated diverticula. This increases the efficiency of the overall process as diverticula which have been filled can be quickly identified.

Suitable marking can be achieved in a variety of ways. For example, a marker "flag" can be provided in the form of an attachment to the filling means and/or anchor. Preferably such a flag is formed of a biodegradable material such as sodium alginate.

Alternatively or additionally, a dye can be used as a marker. The dye can be applied as a spray, or as an injection ie a tattoo. Preferred dyes include 5% methylene blue; Lugol's solution; toluidene blue; 1:4 solution of washable blue ink; 0.1% indigo carmine. A suitable tattoo medium could be an aliquot of sterile Indian ink, diluted 1:100. The use of dyes is within the knowledge of a skilled person.

Preferably, the means for inserting the filling means into a diverticulum is a catheter.

Preferably, the catheter has a recess for receiving the filling means, a release mechanism for releasing the filling means from the catheter and, optionally, injection means for injecting filler material into a diverticulum.

More preferably, the catheter comprises an inner and an outer tube, the inner tube slidably engaging the outer tube and having an injection means at a first end and a part for receiving the filler material at a second end. The outer tube has an end portion outwardly extending from the first end of the inner tube defining the recess for receiving the filling means with the second end of the outer tube being joined to the inner tube by a seal. The release mechanism, in use, reciprocates the outer tube relative to the inner tube. In this preferred arrangement the outer tube sits over the mouth of a diverticulum and acts as a restraining brace while the filling means is inserted into the diverticulum, filled with the filler material, and the injection means removed from the filler means. This prevents application of any undue force to the inside of the diverticulum being plugged, which increases patient compliance and further accelerates the encapsulation of the filling means with body tissue.

Preferably, the seal joining the outer tube to the inner tube has an inlet port for receiving a biocompatible fluid. This enables the Endoscopist to flush any body tissue from the recess restraining the filling means or to wash the inside of a diverticulum prior to insertion of the filling means. Alternatively, the Endoscopist may pass a dye through the inlet port and spray mark the diverticulum which has been filled. This permits the Endoscopist to quickly identify which diverticula have been filled, thus increasing the efficiency of the overall process, with a concomitant decrease in expenditure and an increase in patient compliance.

Preferably, the treatment of diverticular disease comprises. treating one or more bleeding diverticular.

### Description of the drawings

Preferred embodiments of the invention will now be described by way of example, with reference to the accompanying drawings in which:
Figure 1 is a longitudinal cross-section of a preferred balloon;
Figure 1a is an end view of uninflated balloon of Figure 1;
Figure 2 is a partial cross-sectional view of a preferred non-return valve;
Figure 2a is a cross-section through a balloon used in the invention prior to stem inversion;
Figure 3 is a longitudinal cross-sectional view of a preferred balloon having anchor means;
Figure 4 is a cross-sectional view of a preferred anchor of the balloon of Figure 3;
Figure -5 is a plan view of the anchor of Figure 4;
Figure 6 is a longitudinal cross-sectional view of the balloon of Figure 3 inflated inside a diverticulum;
Figure 7 is a partial cross-sectional view of the balloon of Figure 3 mounted at the distal end of preferred delivery means in the form of a catheter;
Figure 8 is a partial cross-sectional view of the balloon of Figure 1 mounted at the distal end of preferred delivery means in the form of a catheter;
Figure 9 is a partial cross-sectional view of a balloon mounted at the distal end of a catheter having a sheath;
Figure 10 is a partial cross-sectional view of a proximal end of preferred delivery means in the form of a catheter; and
Figure 11a and 11b are side and end views; respectively of another preferred anchor;
Figures 12a and 12b are partial cross-sectional side and end views, respectively of the anchor of Figures 11a and 11b mounted at the distal end of a catheter; and
Figure 13a illustrates diverticula of the colon; Figure 13b shows a diverticulum formed by herniation of the intestinal mucosa through the weakened muscular wall at site of arterial penetration on the mesenteric border of the colon; Figure 13c illustrates how faecal matter can accumulate within the diverticulum.

### 1. Inflatable balloon

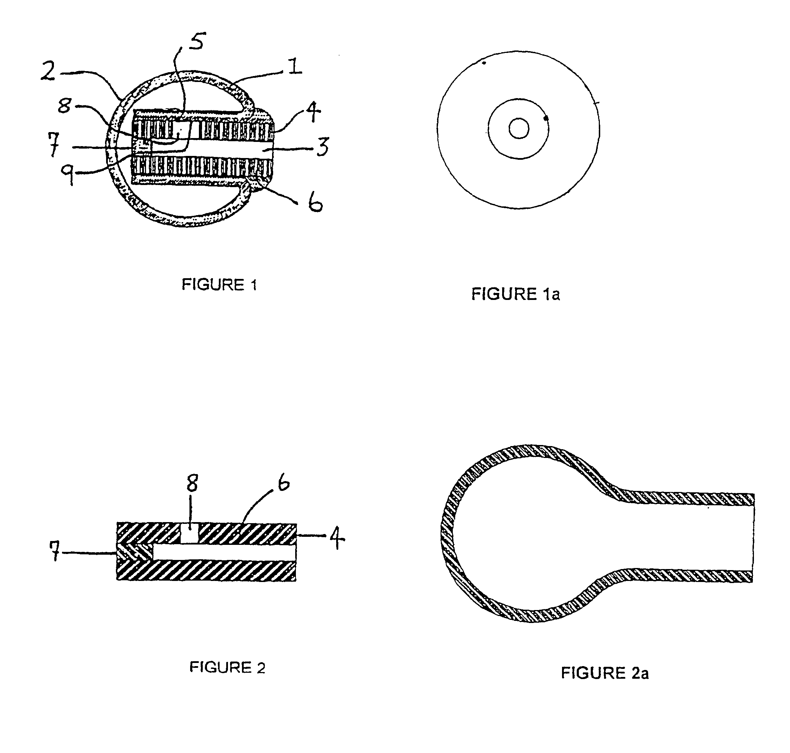

Figure 1 shows a preferred embodiment of the invention comprising a balloon (1) having a coating of a non-metallic haemostatic agent that retards or prevents bleeding (2), and inflation port (3) and a non-return valve (4).

The balloon is preferably made from an elastomeric material, such as a thin silicone polymer, by methods known to those skilled in the art, such as by dip moulding.

In a more preferred embodiment the non-metallic agent is provided as a knitted fabric which is draped over the balloon.

The inflation port (3) and/or non-return valve (4) is preferably made from a silicone polymer and is affixed to the balloon by gluing with a silicone adhesive or other affixing methods which are within the knowledge of a skilled person. The neck (5) of the balloon (1) is turned inside out and placed over the non-return valve (4). The balloon (1) is then glued to the front edge of the non-return valve (4) only so that the neck (5) of the balloon grips the outer wall of the non-return valve (4).

The non-return valve (4), shown in Figures 1 and 2, is made from an elastomeric material, such as a thin silicone elastomer by methods known to those skilled in the art, such as injection moulding.

Preferably, the non-return valve (4) is a tube (6) having a seal (7) sealing one end and an outlet (8) in its side wall. The outlet (8) is covered by the neck (5) of the balloon (1) which grips the outer wall of the valve (4) and functions as a valve cover (9).

The outer surface of the balloon is textured or roughened to increase its effective surface area, thereby promoting tissue growth and encouraging complete encapsulation of the balloon in use. Preferably, the balloon is also coated with an abrasive material. Additionally, or alternatively, the balloon may be coated with an agent to promote further tissue growth and encourage rapid encapsulation. The growth promoting agent may be one or more of a number of bio-absorbable polymers, especially a polyglycolic acid (PGA), or one or more of polylactic acid (PLA), polylactide, cellulose ester, polycaprolactone, polyethylene glycol, polyphosphate ester, collagen, polydioxanone (PDS), trimethylene carbonate (TMC), hyaluronic acid (HLA) and other glycosaminoglycans

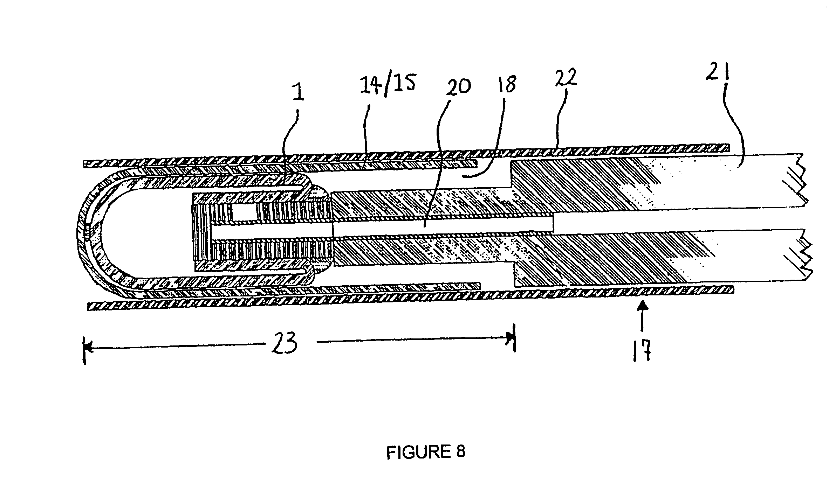

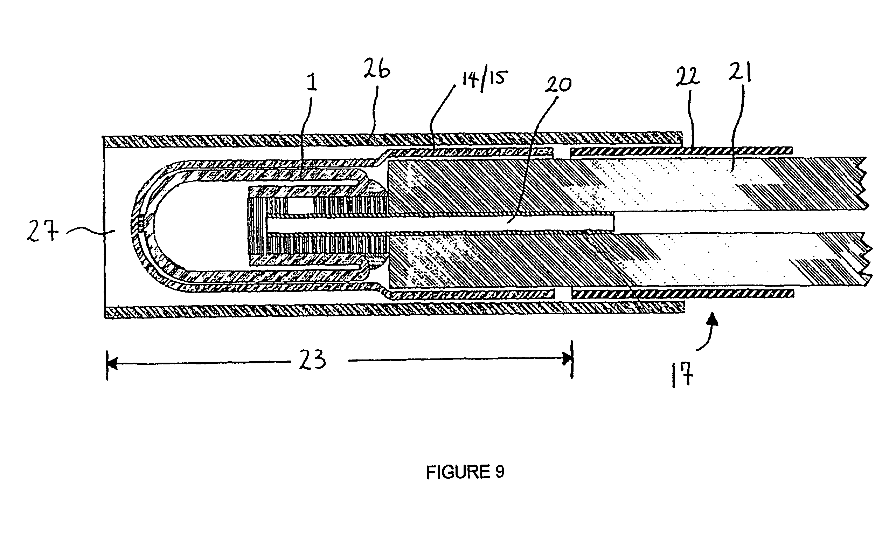

Preferably the balloon is inserted in the end of a catheter, as shown in Figures 8 and 9. This allows the balloon to be delivered into a diverticulum via the working channel of a conventional endoscope.

### 2. Alternative embodiment not forming part of the invention with optional anchor means

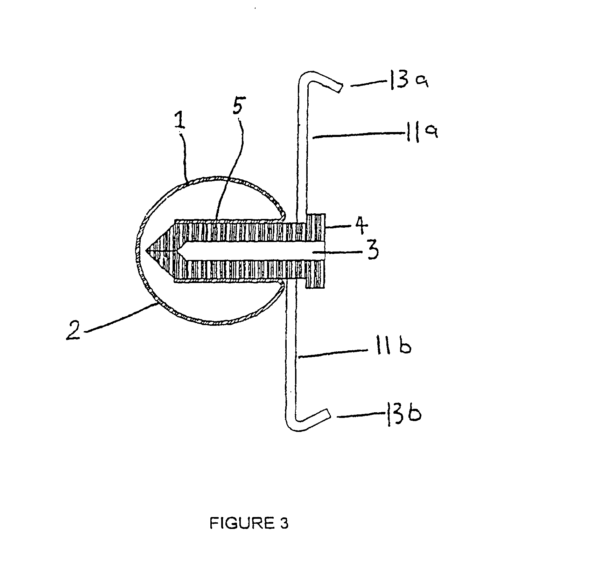

Figure 3 shows an alternative form of a balloon (1) having an alternative inflation port (3) and/or non-return valve (4) in an inflated condition having a preferred anchor (10) in a retaining position.

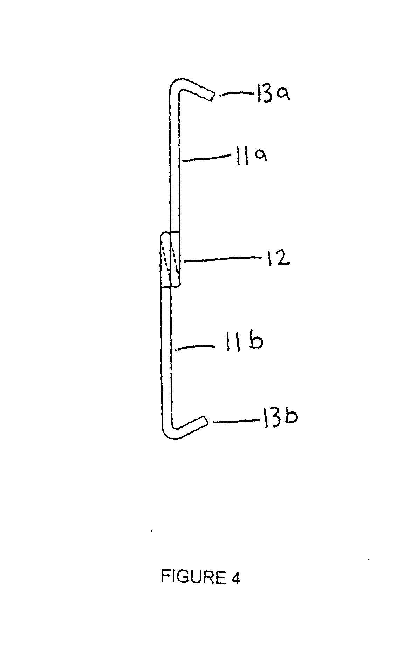

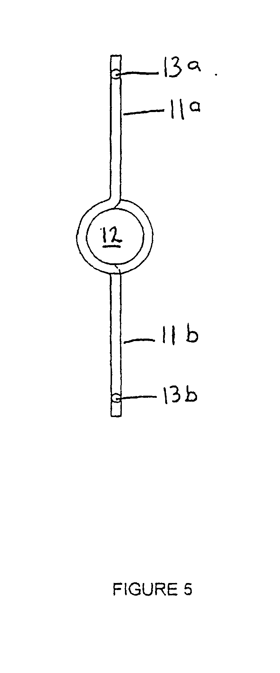

As shown in Figures 3 to 6 the preferred anchor (10) has two arms (11a,11b) which, in the first retaining position shown, extend radially outwards from the balloon (1). As shown in Figures 4 and 5, the two arms (11a,11b) are of equal length and extend radially from diametrically opposed points of a central hub (12). The two arms (11a,11b) have barbs (13a,13b) at the ends furthermost from the central hub.

The anchor (10) may be connected to the inflation port (3), or more preferably, as shown in Figure 3, connected to the combined inflation port (3) and non-return valve (4). The anchor (10) can of course be connected to the balloon by any known technique.

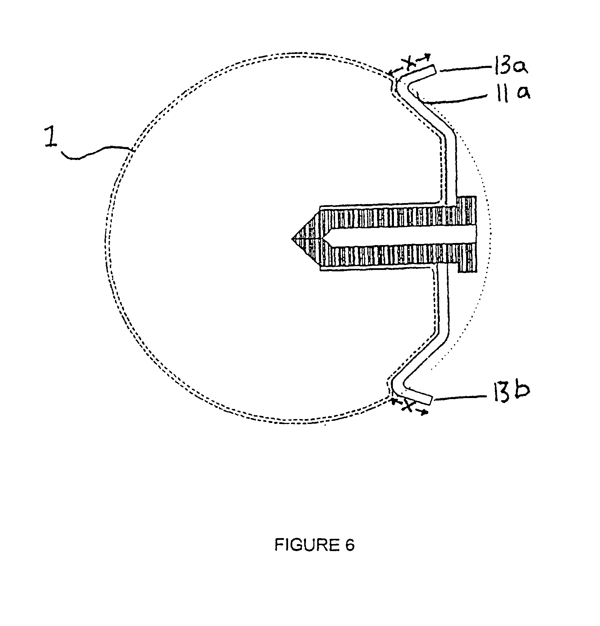

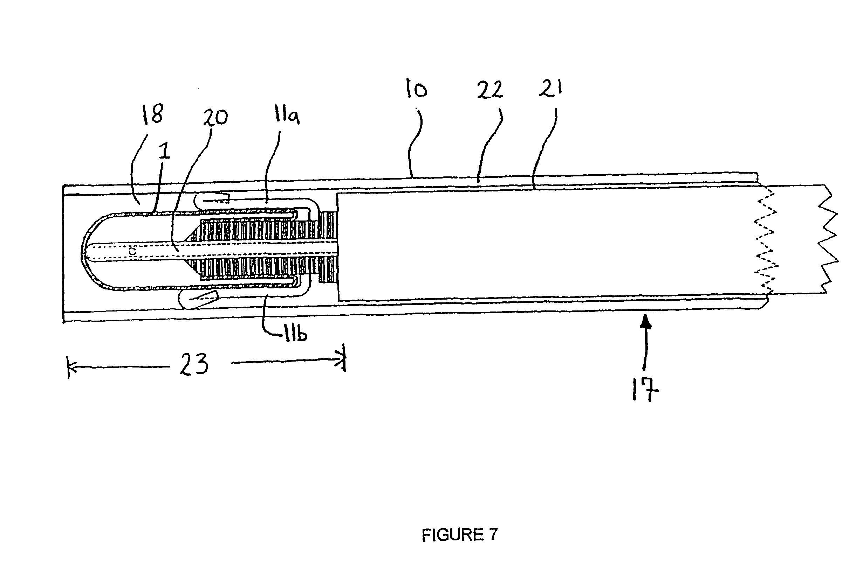

Preferably, the anchor (10) is constructed from the shape memory alloy nitinol which is available from Euroflex Schussler GmbH, Kaiser-Friedrich, Str 7, D-75172, Pforzhelm, Germany. The anchor can be folded from a first retaining position (shown in Figure 6 for example) into a second deformed condition in which it is small enough to allow the balloon to be inserted into a suitable delivery device, as shown in Figure 7.

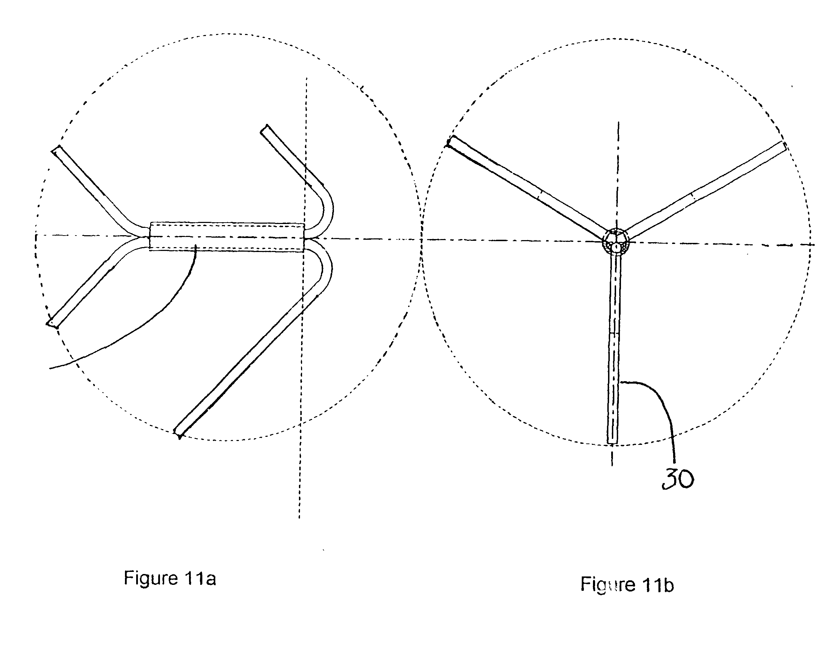

An alternative preferred anchor arrangement (30) is shown in Figures 11a and 11b. The anchor is made from nitinol wire and or nitinol tubing.

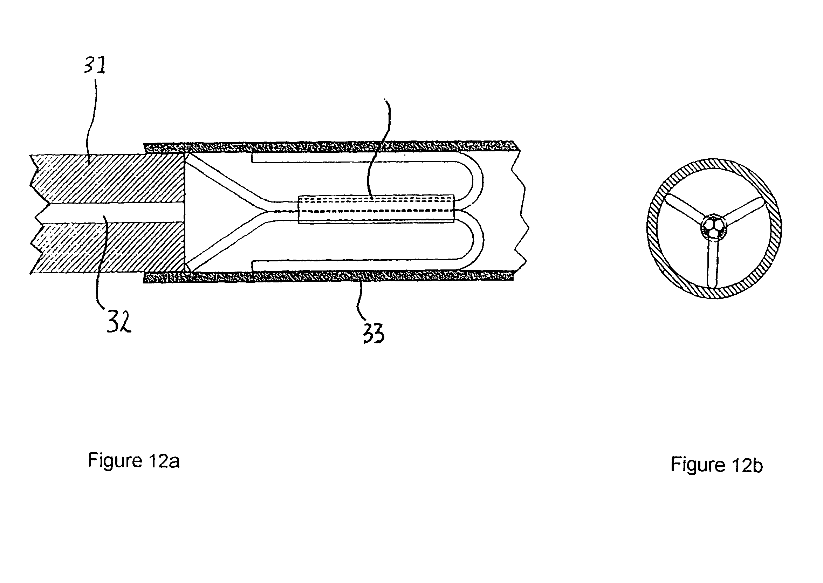

Figures 12a and 12b show the anchor (30) mounted inside the distal end of a preferred catheter having an anchor ejector tube and a central lumen for spraying or injecting a marker dye.

An anchor constructed of nitinol can easily be formed on a jig from a piece of nitinol wire, fixed by a simple heat treatment in a muffle furnace for example, so that the nitinol is set into its first retaining position.

Alternative methods of making the anchor include standard techniques known to those skilled in the art, for example, by stamping from sheet material, from a tube or a combination of these methods.

### Marker Means

Preferably, a flat (not shown) is attached to the anchor and protrudes from the neck of a diverticulum after the anchor has been inserted. More preferably, the flag is constructed from either a water-soluble or biodegradable polymer so that it is quickly absorbed after the procedure. In a particularly preferred arrangement the flag is made of cellulose alginate.

### 3. Non-metallic haemostatic agent that retards or prevents bleeding

According to the present invention, the balloon is associated with a non-metallic agent that prevents or retards bleeding (2). The presence of such a non-metallic (hemostatic) agent, in use, shortens the time needed to stop arterial bleeding of a diverticulum compared to the time needed with a balloon on its own.

Preferably, the non-metallic haemostatic agent (2) is oxidised celulose, calcium alginate, gelatine or collagen, advantageously in the form of a net (14) or knitted textile material (15) that envelopes the balloon (1). The net (14) or knitted textile material (15), is glued to the distal tip of the balloon (1) with a small amount of silicon adhesive, as shown in Figure 8.

Alternatively, the non-metallic haemostatic agent (2) is provided in the form of a flexible film (not shown) that coats the outer surface of the balloon. The balloon (1) is coated with the flexible film by conventional methods.

In a particularly preferred arrangement the net (14), knitted textile material (15), or the flexible film is textured or roughened to increase it effective surface area, thereby promoting tissue growth and encouraging complete encapsulation of the balloon in use.

### 4. Delivery means

As shown in Figures 7, 8 and 9, a preferred delivery means for inserting the balloon into a diverticulum and means for inflating by filling the balloon with a filler material is a catheter (17). The catheter has a recess (18) for receiving the balloon, a release mechanism (not shown) for releasing the balloon (1) from the catheter and an injection means (20) for filling and thereby inflating the balloon (1) with a filler material. As shown in Figures 7 to 9, the catheter comprises two coaxial tubes; an inner tube (21) that is slidably engaged with an outer tube (22).

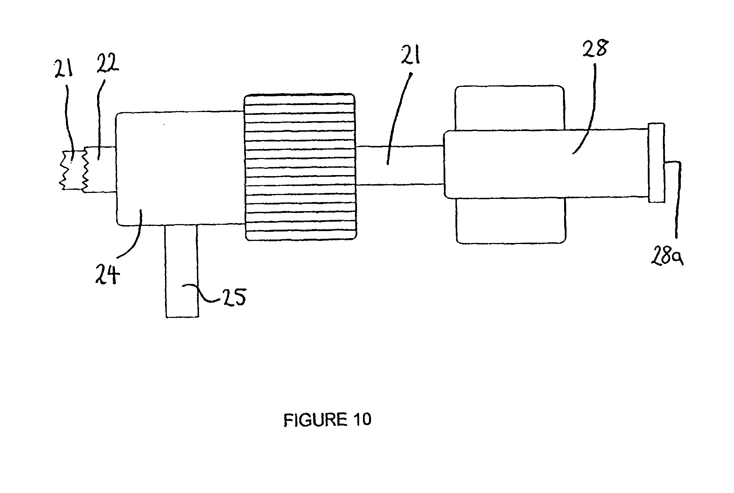

As shown in Figures 7 and 8, the distal end of the outer tube (22) has an end portion (23) that extends outwardly from the distal end of the inner tube (21), thereby defining the recess (18) to receive and constrain the balloon (1) prior to insertion into a diverticulum. As shown in Figure 10, the proximal end of the outer tube (22) is connected to the inner tube (21) by a seal (24), having an inlet port (25) to receive a bio-compatible fluid such as water into the space between the inner and outer tubes. Preferably, the seal (24) is a "Toughy-Borst" connector which is available from Qosina Company of 150-Q Executive Drive, Edgewood, New York, 11217 USA. Preferably, the bio-compatible fluid is used to flush the recess (18) and thereby remove any body tissue, or for washing the inside of a diverticulum, prior to insertion of the balloon (1). Alternatively, or additionally, the fluid may be a dye for marking a diverticulum that has received a balloon (1).

Alternatively, or additionally, as shown in Figure 9, the distal end of the outer tube (22) has a sheath (26) slidably attached thereto. The sheath (26) extends outwardly from the distal end of the outer tube (22) and defines a recess (27) to receive and constrain a balloon prior to insertion into a diverticulum. The sheath (26) permits a larger diameter balloon or a standard size balloon having a thicker hemostatic net (14) or a thicker hemostatic knitted material (15) to be carried by the catheter (17) which, in use, allows diverticulae of varying sizes to be plugged.

The proximal end of the inner tube (21), as shown in Figure 10, terminates with a standard luer pressure tight connector (28) having a stopcock (28a) for receiving a pressure controlled inflation device (not shown) having a monitor for both volume and pressure. Such a pressure tight connector is also available from Qosina Company.

Preferably, the pressure controlled inflation device is a normal 30ml syringe used in standard angioplastic procedures with balloon catheters. The syringe has an accurate graduation for measuring the volume of fluid injected into the balloon. It is also fitted with a pressure gauge that preferably monitors pressure in the region of 0 to 100 kPa (0 to 15 psi). In use, pressure and volume measurements enable the balloon to be fully inflated inside the diverticulum whilst reducing the risk of bursting the diverticulum, which could lead to perforation into the peritoneum.

Preferably, the length of the continuous double tube is greater than 2.25 metres. Preferably the outer tube has an outside diameter of between 7 and 12 French (2.5-4mm), so that it is able to fit inside the working channel of a conventional endoscope.

The distal end of the inner tube (21) terminates in an injection means (20) for filling the balloon with a filler material which is preferably a biocompatible fluid such as water.

The injection means (20) may be permanently sealed to the distal end of the inner tube using standard methods, or be removably sealed to the distal end of the inner tube using standard methods, for example a screw thread type connection. Preferably, the injection means is a nozzle, a tubular member or an injection needle. More preferably, the outer diameter of the injection means (20) is slightly larger than the relaxed inner diameter of the inflation port (3) and/or non-return valve (4) of the balloon. This results in a frictional force between the injection means (20) and inflation port (3) / non-return valve (4) which, in use, retains the balloon (1) on the injection means (20).

At the proximal end of the continuous double tube catheter is the release mechanism (not shown), that moves the inner tube within the outer tube by an amount not less than the length of the inflation device that is mounted at the distal end of the inner tube and constrained by the outer tube.

### 5. Preferred method of plugging a diverticulum

The method described is merely exemplary and not part of the invention. The complete procedure is carried out endoscopically. A preliminary examination is usually carried out to determine the extent of the disease, preferably by a combination of barium x-ray and colonoscopy. The number and approximate position of the diverticulae to be filled will therefore usually be known. Patient preparation is the same as for normal colonoscopy, preferably the endoscope is inserted and the colon inflated.

### Pre-surgical preparation

As shown in Figures 7 to 9, the balloon (1) is mounted onto the injection means (20) at the distal end of the inner tube (21) with the outer tube (22) or the sheath (26) constraining the balloon (1) within the recess (18 and 27, respectively). If the balloon (1) includes an anchor (10), the anchor is folded, preferably longitudinally along the balloon, and the balloon (1) is mounted on the injection means (20), as shown in Figure 7.

Air is removed from the apparatus before commencing the surgical operation by connecting the syringe, typically a 30ml syringe, to the stopcock (28a) of the luer fitting (28), opening the stopcock (28a) and withdrawing the syringe piston. The stopcock (28a) is closed and the syringe removed. The apparatus is now ready for use.

### Surgical procedure

For a standard sized balloon, as shown in Figures 7 and 8, the user checks that the balloon (1) having the hemostatic (i.e. non-metallic haemostatic agent that retards or prevents bleeding) net (14) or hemostatic knitted textile material (15) is constrained within the recess (18) and protected by the outer tube (22) of the catheter (17). The "Toughy-Borst" connector is tightened and the distal end of the double walled catheter (17) is threaded through the working channel of a standard endoscope, the endoscope inserted, the colon inflated, and the balloon delivered to the mouth of a diverticulum using the controllable tip of the endoscope.

Prior to inserting the balloon into the diverticulum, it may be necessary to wash the inside of the diverticulum. This can be carried out by placing the distal end of the outer tube over the mouth of the diverticulum, and passing a bio-compatible fluid, such as water, through the inlet port on the "Toughy- Borst" type seal.

The distal end of the outer tube is presented to the mouth of the diverticulum and the balloon is pushed into the diverticulum by advancing the inner tube.

After the balloon has been completely inserted into the diverticulum, a pressure controlled inflation device, preferably a 30ml syringe containing sterile water with no air bubbles and fitted with a pressure gauge, is attached to the stopcock (28a) of the standard luer fitting (28), the stopcock (28a) opened and the balloon (1) inflated to a specific volume and/or pressure with the sterile water, so that the balloon completely fills the diverticulum. The required volume/pressure for inflation of the balloon can be determined by routine trial and error testing by a skilled person, though it is desirable that the pressure does not exceed 65kPa (10psi) as leakage at the balloon and catheter junction may occur if too much pressure is applied or if the balloon is inflated too quickly. It is exemplary that the inner tube of the delivery means has a total volume of lml and the balloon expands by 1cm in diameter when filled with 0.53 ml of fluid.

The inner tube is then withdrawn to remove the injection means from the inlet port of the balloon and the non-return valve prevents the water from escaping from the balloon. The advantage of withdrawing the inner tube (21) prior to withdrawing the outer tube (22) is that the outer tube acts as a restraining brace, thereby preventing any undue force inside the diverticulum.

When the balloon (1) and anchor (10) is inserted into a diverticulum and the balloon inflated, as shown in Figure 6, the anchor returns to its retaining position in which the barbs (13a,13b) engage the interior wall of a diverticulum and prevent the balloon from being expelled from the diverticulum by the peristaltic movement of the bowel. The barbs of the anchor are arranged so that their penetration of a diverticulum interior wall is limited to the distance X. Herein, such arrangement to limit penetration is termed "abutment means".

In the apparatus provided with anchor means, when a larger diameter balloon or a balloon having a thicker hemostatic net (14) or a thicker hemostatic knitted material (15) is used, as shown in Figure 9, the protective sheath (26) is moved towards the proximal end of the double walled catheter (17) to expose the top, preferably 3 to 4mm, of the balloon (1). The "Toughy-Borst" connector is tightened and the tip of the balloon is inserted into the mouth of a standard endoscope and the double walled catheter, preferably a first length of 75mm, is pushed through the sheath (26) and threaded through the working channel of the endoscope. The sheath (26) is withdrawn along the length of the catheter (17) to the proximal end thereof. The remainder of the catheter is threaded through the channel of the endoscope, the endoscope inserted, the colon inflated, and the balloon delivered to the mouth of a diverticulum using the controllable tip of the endoscope.

The balloon is pushed into the diverticulum by advancing the inner tube. After the balloon has been completely inserted it is inflated by the same procedure as described previously.

Preferably, the filled diverticulum is marked by passing a dye spray down through the inlet port of the "Toughy-Borst" seal.

Once a diverticulum has been plugged with a balloon, the apparatus is reloaded with a separate balloon and the procedure is repeated as required. Hence, the balloon of the apparatus of the invention remains inside the patient and can therefore be said to be consumed in the treatment method since it is not available for any subsequent uses.

### 6. Alternative preferred filling means

### (i) Curable filler material

Curable filler material is provided as a non-solid form e.g. liquid, gel, or paste and will cure or set to a solid form after insertion into a diverticulum. Preferably the material sets very quickly, so that the diverticula plugging procedure can proceed quickly.

Non-exhaustive examples of curable material include silicone elastomers, polyurethane foams and elastomers, epoxy resins etc.

Skilled person will appreciate that curable materials can be provided in the form of two or more discrete substances which cure on mixing.

Curing may be effected by a variety of means including solvent evaporation, UV or visible light irradiation or heating. Preferably the curable material which cures at human body temperature.

### (ii) Fibrous filler material

Fibrous filler material such as textile fibres or threads can be used to fill a diverticulum. Preferably the fibre or thread is coated with an adhesive material before or after insertion into a diverticulum.

### (iii) Resiliently deformable member

Filling means in the form of a resiliently deformable member represent another embodiment which however is not part of the invention.

In use, the member is deformed from a retaining position into a second smaller position and inserted into a diverticulum. Once inside the diverticulum the deformable member returns to its retaining position so that it is retained within the diverticulum.

Preferably, the deformable member is an elastomeric member e.g. foamed plastic such as foamed silicone or foamed polyurethane which can be compressed and inserted into a diverticulum. When the compression force is released the member expands into its uncompressed retaining position and thereby fills the diverticulum.

The deformable member may also comprise a member formed from a shape memory alloy such as nitinol, which can be folded for insertion and expand to a predetermined retaining position in response to a patient's body heat, for example

### 7. Alternative preferred delivery means

It will be appreciated that it may be necessary to use different delivery means depending on the filling means used according to the invention.

If filling means (i) or (ii) are used in combination with anchor means, the delivery means will comprise a two-part system, one for delivering the anchor and the other for delivering the filler material. A plurality of anchors can be mounted in a magazine at the distal end of the catheter.

The catheter allows the anchors to be fed one at a time into a delivery nozzle at the distal end. The delivery nozzle can be withdrawn to release the anchor, after the distal end of the catheter is placed into the neck of the diverticulum. In this way a number of anchors may be inserted into multiple diverticula without the need to withdraw the catheter from the endoscope after each insertion.

The filler is injected into the diverticula using an injection catheter and syringe.

The catheter is multi-lumen when using a two-part mix curable material.

The two lumen come together at the distal end of the catheter and terminate into a single nozzle where the contents of each lumen are mixed. At the proximal the material is injected via a two lumen syringe.

According to the type of filler used, curing can be carried out via another lumen of the injection catheter, or by a separate catheter. For example a fibre optic carrying UV or visible light could be delivered via a separate lumen.

### Delivery catheter - anchor

As shown in Figures 12a and 12b, a separate anchor delivery catheter may consist of an anchor ejector tube (31) and a central lumen (32) for spraying or injecting a marker dye. A number of folded anchors can be loaded into the distal end of the outer tube (33).

At the proximal end of the catheter is a mechanism which can withdraw the outer tube over the anchor ejector tube by an indexed amount equal to the length of one folded anchor.

The distal end of the catheter is inserted into the neck of the diverticula and then the outer tube is withdrawn by an indexed amount. This will release one anchor into the diverticulum, allowing it to expand to its full size. Preferably, a flag is attached to the proximal end of the folded anchor, and is folded behind it. Thus, the flag will protrude from the neck of the diverticulum in use.

### Delivery Catheter - Filler

The design of the filler catheter varies according to the material type of the filler. In its simplest form it is a single lumen tube with an injection nozzle at the distal end. The filler material is injected into the diverticulum by an indexing pump at the proximal end of the catheter.

If the filler material is a two part resin, then a two lumen catheter is used with a mixer nozzle at the distal end.

### Other preferred methods: using a separate anchor and non-solid curable and/or fibrous filler materials

Again, the method described is merely exemplary and does not form part of the invention. The first part of the procedure is to insert the anchors. The loaded insertion catheter is first introduced into the colon via the working lumen of the endoscope. These are inserted into the diverticula from an anchor magazine insertion arrangement on the distal end of the insertion catheter. Soluble or biodegradable flags may be left protruding from the diverticula neck to show which have had an anchor inserted.

When all the anchors have been inserted the anchor insertion catheter is withdrawn from the endoscope.

As an alternative to the flag, a central lumen of the anchor insertion catheter may be used to spray a dye around the neck of the filled diverticula. To prevent the dye leaking proximally, the proximal end of the outer tube is terminated in a seal of the "Toughy-Borst" type.

The filler catheter is now introduced into the colon via the endoscope lumen. Using the flags (or dye spray), as a guide, each diverticula is injected with the filler material, so that it is completely full. The material will be in the form of a gel, or a past or a glue coated textile fibre, so that, it will not be expelled before it is cured, due to the peristaltic action of the colon.

If the material is self curing, then all that remains is to leave the colon inflated until the material is set. If the material requires an additional curing means, then this is now applied.

## Claims

1. Apparatus comprising a delivery means and a balloon (1) connected to the delivery means **characterised in that**, when the balloon is deflated, the deflated balloon has a diameter dimensioned to permit entry through the neck of a diverticulum and wherein the balloon is associated with a non-metallic haemostatic agent that retards or prevents bleeding (2).

2. Apparatus as claimed in Claim 1 wherein the balloon (1) is a single balloon.

3. Apparatus as claimed in Claim 1 or 2 wherein the balloon (1) includes means to prevent deflation.

4. Apparatus as claimed in Claim 1, 2 or 3 wherein the balloon (1) is releasably connected to the delivery means.

5. Apparatus as claimed in any preceding claim wherein the non-metallic agent that retards or prevents bleeding (2) is selected from one or more of oxidised cellulose, carboxymethylated cellulose, calcium alginate, gelatine or collagen.

6. Apparatus as claimed in any preceding Claim wherein the non-metallic haemostatic agent that retards or prevents bleeding (2) is provided in the form of a net or a knitted textile material that envelopes the balloon or in the form of a flexible film that coats the outer surface of the balloon (1).

7. Apparatus as claimed in Claim 6 wherein the net or knitted textile material is fixed to the balloon (1).

8. Apparatus as claimed in any preceding Claim wherein the balloon (1) is made from silicone rubber.

9. Apparatus as claimed in any preceding Claim wherein the balloon (1) is impermeable to liquid but permeable to gas.

10. Apparatus as claimed in any preceding Claim wherein the balloon (1) is an inflatable balloon having an inflation port (3).

11. Apparatus as claimed in Claim 10 wherein the inflation port (3) includes a non-return valve (4) to prevent deflation of the balloon (1).

12. Apparatus as claimed in Claim 11 wherein the non-return valve (4) comprises a tube (6) sealed (7) at one end having an outlet (8) in the side wall, the outlet (8) being releasably sealed with a valve cover (9).

13. Apparatus as claimed in Claim 12 wherein the balloon neck (5) is the valve cover (9).

## Patentansprüche

1. Vorrichtung mit einem Setzmittel und einem Ballon (1), der an das Setzmittel angeschlossen ist, **dadurch gekennzeichnet, dass** dann, wenn der Ballon abgeblasen ist, der abgeblasene Ballon einen Durchmesser von solchen Abmessungen hat, die ihm den Zugang durch den Hals eines Divertikels ermöglichen und dass der Ballon mit einem nichtmetallischen blutstillenden Mittel verbunden ist, das Blutungen (2) hemmt oder verhindert.

2. Vorrichtung nach Anspruch 1, wobei der Ballon (1) ein einzelner Ballon ist.

3. Vorrichtung nach Anspruch 1 oder 2, wobei der Ballon (1) Mittel umfasst, die sein Abblasen verhindern.

4. Vorrichtung nach Anspruch 1, 2 oder 3, wobei der Ballon (1) lösbar an das Setzmittel angeschlossen ist.

5. Vorrichtung nach einem der vorangehenden Ansprüche, wobei das nicht-metallische Mittel, das Blutungen (2) hemmt oder verhindert, aus oxidierter Cellulose, carboxymethylisierter Cellulose, Calciumalginat, Gelatine und/oder Collagen ausgewählt ist.

6. Vorrichtung nach einem der vorangehenden Ansprüche, wobei das nicht-metallische blutstillende Mittel, das Blutungen (2) hemmt oder verhindert in der Form eines Netzes oder eines geknüpften Textilmaterials vorgesehen ist, das den Ballon umgibt, oder in der Form eines flexiblen Films, der die äußere Oberfläche des Ballons (1) bedeckt.

7. Vorrichtung nach Anspruch 6, wobei das Netz oder das geknüpfte Textilmaterial an dem Ballon (1) fixiert ist.

8. Vorrichtung nach einem der vorangehenden Ansprüche, wobei der Ballon (1) aus Silikongummi besteht.

9. Vorrichtung nach einem der vorangehenden Ansprüche, wobei der Ballon (1) undurchlässig für Flüssigkeit aber durchlässig für Gas ist.

10. Vorrichtung nach einem der vorangehenden Ansprüche, wobei der Ballon (1) ein aufblasbarer Ballon mit einer Aufblasöffnung (3) ist.

11. Vorrichtung nach Anspruch 10, wobei die Aufblasöffnung (3) ein Rückschlagventil (4) umfasst, um das Abblasen des Ballons (1) zu verhindern.

12. Vorrichtung nach Anspruch 11, wobei das Rückschlagventil (4) ein Rohr (6) aufweist, das an einem Ende abgedichtet ist (7) und einen Auslass (8) in der Seitenwandung aufweist, wobei der Auslass (8) nachgiebig mit einem Ventildeckel (9) abgedichtet ist.

13. Vorrichtung nach Anspruch 12, wobei der Ballonhals (5) der Ventildeckel (9) ist.

## Revendications

1. Appareil comprenant des moyens de distribution et un ballonnet (1) relié aux moyens de distribution **caractérisé en ce que**, lorsque le ballonnet est dégonflé, le ballonnet dégonflé a un diamètre dimensionné de façon à permettre l'entrée à travers le col d'un diverticule et dans lequel le ballonnet est associé à un agent hémostatique non métallique qui retarde ou prévient les hémorragies (2).

2. Appareil selon la revendication 1 dans lequel le ballonnet (1) est un ballonnet simple.

3. Appareil selon la revendication 1 ou 2 dans lequel le ballonnet (1) comprend des moyens pour empêcher le dégonflage.

4. Appareil selon la revendication 1, 2 ou 3 dans lequel le ballonnet (1) est relié de façon amovible au moyen de distribution.

5. Appareil selon l'une quelconque des revendications précédentes dans lequel l'agent non métallique qui retarde ou prévient les hémorragies (2) est choisi à partir de un ou plusieurs des produits suivants : cellulose oxydée, cellulose carboxyméthylée, alginate de calcium, gélatine ou collagène.

6. Appareil selon l'une quelconque des revendications précédentes dans lequel l'agent hémostatique non métallique qui retarde ou prévient les hémorragies (2) se présente sous la forme d'un filet ou d'un matériau textile à mailles qui enveloppe le ballonnet ou sous la forme d'un film souple qui recouvre la surface externe du ballonnet (1).

7. Appareil selon la revendication 6 dans lequel le filet ou le matériau textile à mailles est fixé au ballonnet (1).

8. Appareil selon l'une quelconque des revendications précédentes dans lequel le ballonnet (1) est en caoutchouc de silicone.

9. Appareil selon l'une quelconque des revendications précédentes dans lequel le ballonnet (1) est imperméable aux liquides mais perméable au gaz.

10. Appareil selon l'une quelconque des revendications précédentes dans lequel le ballonnet (1) est un ballonnet gonflable ayant un orifice de gonflage (3).

11. Appareil selon la revendication 10 dans lequel l'orifice de gonflage (3) comprend un clapet de non-retour (4) pour empêcher le dégonflage du ballonnet (1).

12. Appareil selon la revendication 11 dans lequel le clapet de non-retour (4) comprend un tube (6) fermé hermétiquement (7) à une extrémité ayant un orifice de sortie (8) dans la paroi latérale, l'orifice de sortie (8) étant scellé de façon amovible avec un couvercle de clapet (9).

13. Appareil selon la revendication 12 dans lequel le col du ballonnet (5) est le couvercle de clapet (9).
